# EUROPEAN PATENT SPECIFICATION

(11) **EP 0 677 508 B1**
(45) Date of publication and mention of the grant of the patent: **07.01.1998**
(21) Application number: 95103596.3
(22) Date of filing: 13.03.1995
(51) Int. Cl.: C07C 227/42

(54) **Process for crystallization of L-phenylalanine monomethyl sulfate**
Verfahren zur Kristallisierung von L-Phenylalaninmonomethylsulfat
Procédé pour cristalliser le monométhyl sulfate de L-phénylalanine

(30) Priority: 12.04.1994 JP 73248/94
(43) Date of publication of application: 18.10.1995
(73) Proprietor: Ajinomoto Co., Inc., Tokyo 104 (JP)
(72) Inventor: Hijiya, Toyoto, c/o Central Res. Laboratories, Kawasaki-ku, Kawasaki-shi, Kanagawa-ken (JP); Sugiyama, Naoko, c/o Central Res. Laboratories, Kawasaki-ku, Kawasaki-shi, Kanagawa-ken (JP); Takemoto, Tadashi, c/o Central Res. Laboratories, Kawasaki-ku, Kawasaki-shi, Kanagawa-ken (JP)
(74) Representative: Strehl Schübel-Hopf Groening & Partner

(56) References cited:
- EP-A- 0 612 717
- US-A- 4 399 304

## Description

This invention relates to a process for recovering L-phenylalanine (hereinafter abbreviated to L-Phe) as the monomethyl sulfate salt thereof in a high yield.

### DESCRIPTION OF PRIOR ART

L-Phenylalanine and its methyl ester, L-phenylalanine methyl ester (hereinafter abbreviated to L-PM) are important as raw materials for peptide synthesis. Particularly, there is a great demand for them as raw materials for a dipeptide sweetener, α-L-aspartyl-L-phenylalanine methyl ester (hereinafter abbreviated to α-APM).

Formerly, various processes for the production of α-APM have been studied. The known industrial processes include, for example, a process which comprises reacting N-protected L-aspartic anhydride with L-PM (US Patent 3786039), a process which comprises the enzymatic condensation of N-benzyloxycarbonyl-L-aspartic acid and L-PM (Japanese Patent Laying-Open No. 92729/1978).

In order to obtain L-PM used herein, a method is often employed, wherein L-Phe is esterified with methanol in the presence of an inorganic acid such as hydrochloric or sulfuric acid, and the resulting acidic reaction solution is neutralized with a suitable base in the presence of water, then the released L-PM is extracted with a water-immiscible organic solvent such as toluene, because L-PM of high purity can be obtained. According to this process, there exist dissolved L-Phe, which remains unreacted in the extract aqueous phase after esterification, and L-Phe, which is produced by decomposition of L-PM upon neutralization and extraction.

L-Phe is a relatively expensive material. Accordingly, it is industrially important to recover L-Phe which fails to be converted to the objective material, and to reuse it as a raw material. L-Phe in the above extract aqueous phase can, for example, be recovered as L-phenylalanine monomethyl sulfate salt (hereinafter abbreviated to L-Phe·MeSO₄H salt) (Japanese Patent Application No. 36881/1993). Since in such recovery L-Phe·MeSO₄H salt, L-tyrosine and D-phenylalanine, which are difficult to efficiently remove as impurities, can be removed with high selectivity by a simple operation, it is possible to obtain L-Phe·MeSO₄H salt of extremely high optical purity from the crystallization system of L-Phe of low optical purity and monomethyl sulfate (hereinafter abbreviated to MeSO₄H salt). Thus, this process is excellent as an industrial recovery of L-Phe.

For example, L-Phe·MeSO₄H salt may be separated by concentration of the extract aqueous phase under acidic conditions when sulfuric acid is used as an acid catalyst. For efficient recovery, however, said aqueous phase should be concentrated to 1/2 to 1/3 of the volume. Such concentration requires a large amount of energy, decreasing the merit of the L-Phe recovery.

When L-Phe·MeSO₄H salt crystals containing impurities are purified by recrystallization from water or slurry-washing, these crystals, highly soluble in water, may be lost in the mother liquor, resulting in reduced yield.

### PROBLEM TO BE SOLVED BY THE INVENTION

The problem to be solved by the invention is to establish a process for crystallization of L-Phe·MeSO₄H salt wherein the solubility of L-Phe·MeSO₄H salt is reduced to improve the yield of the crystallization.

### MEANS TO SOLVE THE PROBLEM

The present inventors have studied intensively to solve the above problems. Surprisingly, we have found that solubility of L-Phe·MeSO₄H salt is reduced remarkably when L-Phe·MeSO₄H salt is recrystallized in the presence of a certain salt. Moreover, when L-Phe·MeSO₄H salt is crystallized from said extract aqueous phase, the solubility is reduced upon addition of such salt. Further, the addition of such salt will provide L-Phe·MeSO₄H salt separated at high yield without increasing the concentration degree of the extract aqueous phase. Thus, we have attained the present invention.

Such salts to be added include, for example, alkali metal halides, such as NaCl, KCl; alkaline earth metal halides such as CaCl₂; alkali metal sulfates such as Na₂SO₄; alkali metal salts of organic acids such as CH₃CO₂Na, CH₃SO₄Na; ammonium halides such as NH₄Cl, etc.

When these salts exist at a higher concentration, their effect to reduce the solubility of L-Phe·MeSO₄H salt becomes greater. When NaCl is used as the cheapest salt for recrystallization or slurry washing of L-Phe·MeSO₄H salt, the effect of the present invention is obviously attained by the existence of the salt at the concentration of 1 g/dl or more, desirably 3 g/dl or more. When the salt is crystallized from said extract aqueous layer, there already exists the salt produced from the base used for neutralization of the esterified reaction solution and the mineral acid such as hydrochloric acid or sulfuric acid which is added for acidification of the extract aqueous layer. In this case, the salt is further added to greatly decrease solubility of L-Phe·MeSO₄H salt. In both cases, however, it is not economical to add a greater amount of salts than required. Further, when the salt is added in an amount exceeding its own solubility, the separated L-Phe·MeSO₄H salt may be contaminated with the added salt. Accordingly, the upper limit of the salt to be added is desirably equal to its solubility. Of course, the addition of salts in an amount beyond its solubility will provide a sufficient effect of the present invention.

Such salt may be used alone or in a combination of two or more of them.

The stage of the addition of such salt may be before or after pH adjustment of the extract aqueous phase, and before or after concentration of the aqueous phase. However, salts which are not neutral should be added before pH adjustment, because the pH may be changed by the addition of such salts. L-Phe·MeSO₄H salt may be recrystallized in the same way.

These salts may be added as solids or in liquid form. "Liquid form" used herein means the state wherein these salts are dissolved in water or are present as a reaction solution in the process for production of α-APM containing these salts. Such reaction solution of the above process comprises, for example:
(i) a mother liquor obtained by hydrolyzing the side flow obtained from production of α-APM under acidic conditions with a mineral acid such as hydrochloric acid, adjusting the pH of the resulting solution around 6 by adding an aqueous alkali solution such as aqueous sodium hydroxide to precipitate L-Phe and removing the L-Phe crystals;
(ii) a mother liquor after removal of L-Asp crystals separated by adjustment of the pH around 3 by addition of acid such as hydrochloric acid to the mother liquor of (i);
(iii) a mother liquor after removal of α-APM crystals separated upon neutralization of α-APM hydrochloride crystals with an aqueous alkali such as sodium carbonate.

Instead of adding preformed salts, the corresponding acid component and alkali component may be separately added and neutralized in a crystallization solution to form salts. For example, hydrochloric acid is added to the crystallization solution, to which is added the equimolar amount of sodium hydroxide, to form sodium chloride.

### BRIEF EXPLANATION OF DRAWINGS

Fig.1 shows the powder X-ray diffraction pattern of the crystal obtained in the Production Example (CuKa ray).

Fig.2 shows the IR chart of the crystal obtained in the Production Example (KBr method).

Fig.3 shows the NMR chart of the crystal obtained in the Production Example (solvent: heavy water).

### EXAMPLES

The present invention will be illustrated in detail in the following examples.

### Example 1

To L-Phe·MeSO₄H salt crystals (10 g) suspended in water (20 ml) were added salts shown in Table 1 (2 g, each). Subsequently, the pH was adjusted to 1.6 - 1.8, then the solution was stirred at 20 °C overnight. After suction filtration, the concentration of L-Phe in the mother liquor was analyzed by an amino acid analyzer. The results are shown in Table 1.

**Table 1**

| Salts added | Concentration of L-Phe in mother liquor (g/dl) |
|---|---|
| None | 11.8 |
| NaCl | 5.8 |
| KCl | 7.1 |
| CaCl₂ | 9.2 |
| NH₄Cl | 3.1 |
| Na₂SO₄ | 9.7 |
| CH₃CO₂Na | 8.6 |
| CH₃SO₄Na | 6.5 |

### Example 2

An esterification was conducted using L-Phe, sulfuric acid and methanol. The reaction solution was neutralized with 15 % aqueous Na₂CO₃, then the generated L-PM was extracted with toluene to give 1,000 ml of extract aqueous layer. According to the results of analysis using an amino acid analyzer, the layer contained 7.7 g of L-Phe. After addition of L-Phe (12 g), the pH was adjusted to 1 with sulfuric acid, followed by concentration under reduced pressure to 770 ml. NaCl (15.2 g) was added to 256 ml of this concentrate, and stirred at 5 °C overnight to crystallize, then the crystals were separated by filtration under reduced pressure. The concentration of L-Phe in the separated mother liquor was 0.43 g/dl, and the yield of crystallization as L-Phe was 85.6 %.

### Comparative Example 1

The procedure of Example 2 was repeated using the concentrate obtained in Example 2 (256 ml), except that NaCl was not added. The concentration of L-Phe in the separated mother liquor was 0.81 g/dl and yield of crystallization as L-Phe was 70.2 %.

### Example 3

In the same manner as in Example 2, 555 ml of the concentrate was obtained (concentration degree: 1.8 times based on the extract aqueous layer). This concentrate (139 ml) was crystallized at 5 °C overnight and the crystals were separated. The concentration of L-Phe in the mother liquor was 0.36 g/dl and yield of crystallization as L-Phe was 92.4 %.

Water (29 ml) was added to the same concentrate (139 ml) to reduce concentration degree (concentration degree: 1.5 times based on the extract aqueous layer). After a similar crystallization, the concentration of L-Phe in the mother liquor was 0.39 g/dl and yield of crystallization as L-Phe was decreased to 89.4 %.

Similarly, water (29 ml) was added to the same concentrate (139 ml) to reduce concentration degree, to which was further added NaCl (13 g) for the crystallization. The concentration of L-Phe in the mother liquor was reduced to 0.26 g/dl, while yield of crystallization as L-Phe was increased to 92.8 %, which is almost the same as that obtained with the solution concentrated to 1.8.

### Example 4

The mother liquor obtained by crystallization of α-APM hydrochloride was heated at 105 °C for 7 hours and hydrolyzed, then adjusted to pH 5.6 with 48 % aqueous NaOH and cooled. The crystallized L-Phe was separated. The mother liquor was adjusted to pH 3.0 with 35 % HCl, then L-aspartic acid was separated. This separated mother liquor contained L-Phe and NaCl at the concentration of 1.1 g/dl and 22.2 g/dl, respectively.

This separated mother liquor (220 ml) was added to the extract aqueous layer (1,150 ml, containing 8.88 g of L-Phe) obtained in the same manner as in Example 2, to which was further added L-Phe (12.4 g), then pH was adjusted to 1.0 with sulfuric acid. After concentrating under reduced pressure to 640 ml, stirring was continued at 5 °C for 3 hours. The separated crystals were removed by filtration to give 61.6 g of crystals and 565 ml of mother liquor. The crystals contained 21.6 g of L-Phe. The concentration of L-Phe in the mother liquor was 0.21 g/dl. Crystallization yield of L-Phe was 94.8 %.

### PRODUCTION EXAMPLE OF L-Phe·CH₃SO₄H

CH₃SO₄Na (13.4 g, 0.1 mol) and L-Phe (4.13 g, 25 mmol) were added to water (35 ml) and adjusted to pH=0 with sulfuric acid, which was heated and dissolved at about 70 °C, then cooled. The separated crystals were removed by suction filtration, washed with a small amount of chilled water, and dried under reduced pressure.

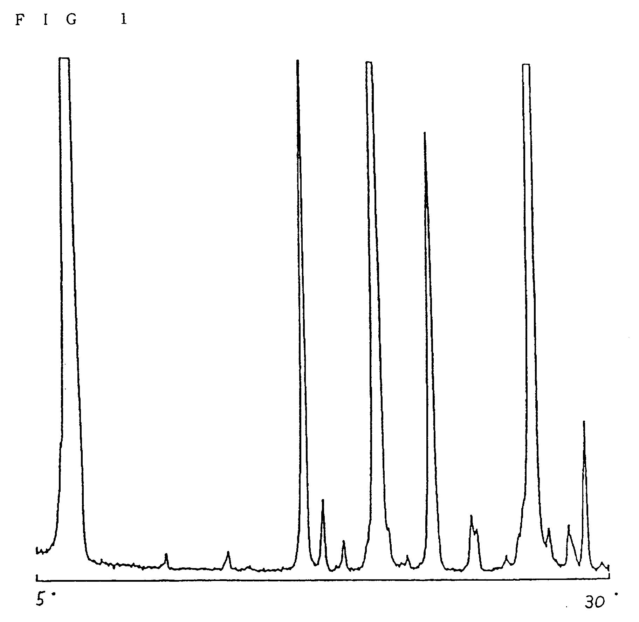

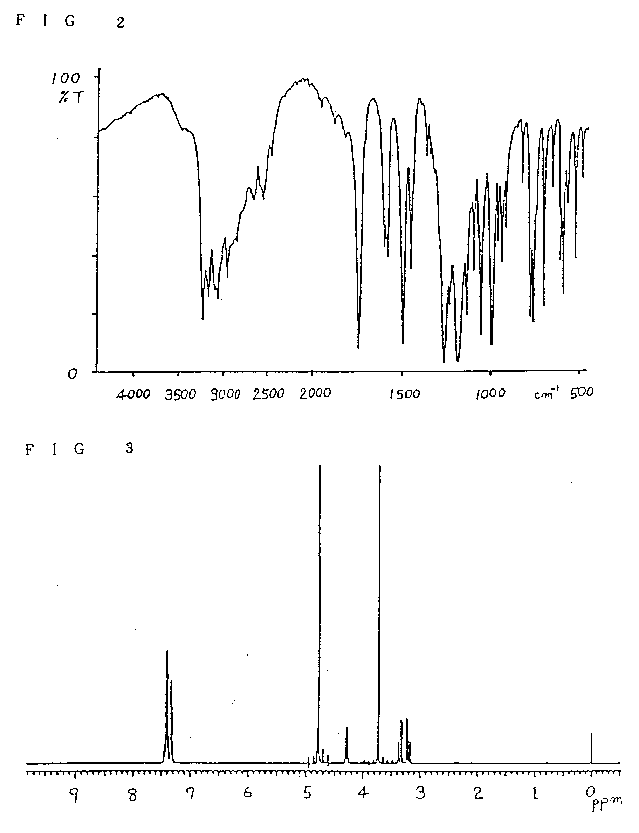

Yield, 5.78 g. The crystals were identified as L-Phe·CH₃SO₄H according to various measurement of physical properties. The results of the measurement are shown in Table 2 and Figures 1 - 3.

**Table 2**

| Composition | Calcd. | Found |
|---|---|---|
| Carbon Content | 43.3 % | 43.0 % |
| Nitrogen Content | 5.1 % | 5.1 % |
| Sulfur Content | 11.6 % | 11.2 % |
| L-Phe | 59.5 % | 60.2 % |
| CH₃SO₄H | 40.4 % | 41.3 % |
| Water Content | 0.0 % | 0.1 % |
| Solubility in solvent | Water | Readily soluble |
| | Methanol | Soluble |
| | Acetone | Almost insoluble |
| | Ether | Almost insoluble |
| Property of aqueous solution | | Acidic |
| Color reaction | Purplish red with ninhydrin | |
| m.p. | 194.4 -195.2 °C (decomp.) | |
| Calculated data of the composition was for CH₃SO₄H·L-Phe | | |

### ADVANTAGE OF THE INVENTION

According to the method of the present invention, a simple operation, i.e., addition of salts during the crystallization of L-phenylalanine monomethyl sulfate will improve the crystallization yield.

## Claims

1. A process for crystallizing L-phenylalanine monomethyl sulfate from an aqueous solution containing L-phenylalanine and monomethyl sulfate, wherein the crystallization is carried out in the presence of an alkali metal or alkaline earth metal or ammonium salt of an inorganic or organic acid in the aqueous solution.

2. A process according to claim 1, wherein the aqueous solution containing L-phenylalanine and monomethyl sulfate is that obtained by a process wherein L-phenylalanine and methanol are esterified in the presence of sulfuric acid, then the reaction solution is neutralized with a base in the presence of water, and the resulting L-phenylalanine methyl ester is extracted with an organic solvent and the layers are separated, and the resulting aqueous phase is acidified to give the aqueous solution.

## Patentansprüche

1. Verfahren zur Kristallisation von L-Phenylalaninmonomethylsulfat aus einer wäßrigen Lösung, die L-Phenylalanin und Monomethylsulfat enthält, wobei die Kristallisation in Anwesenheit eines Alkalimetall-, eines Erdalkalimetall- oder eines Ammoniumsalzes einer anorganischen oder organischen Säure in der wäßrigen Lösung durchgeführt wird.

2. Verfahren gemäß Anspruch 1, wobei die wäßrige Lösung, die L-Phenylalanin und Monomethylsulfat enthält, durch ein Verfahren erhalten wurde, bei dem L-Phenylalanin und Methanol in Anwesenheit von Schwefelsäure verestert werden, danach die Reaktionslösung mit einer Base in Anwesenheit von Wasser neutralisiert wird, der erhaltene L-Phenylalaninmethylester mit einem organischen Lösungsmittel extrahiert und die Schichten getrennt werden, und die erhaltene wäßrige Phase unter Bildung einer wäßrigen Lösung angesäuert wird.

## Revendications

1. Procédé pour cristalliser le monométhyl sulfate de L-phénylalanine à partir d'une solution aqueuse contenant de la L-phénylalanine et du monométhyl sulfate, dans lequel la cristallisation est effectuée en présence d'un sel d'un métal alcalin, d'un métal alcalino-terreux, ou d'ammonium, d'un acide organique ou minéral, dans la solution aqueuse.

2. Procédé selon la revendication 1, dans lequel la solution aqueuse qui contient la L-phénylalanine et le monométhyl sulfate est celle obtenue par un procédé dans lequel de la L-Phenylalanine et du méthanol sont estérifiés en présence de l'acide sulfurique; puis la solution réactionnelle est neutralisée avec une base en présence de l'eau, et l'ester méthylique de L-phénylalanine résultant est extrait par un solvant organique, et les phases sont séparées, et la phase aqueuse résultante est acidifiée pour donner la solution aqueuse.
